# EUROPÄISCHE PATENTANMELDUNG

(11) **EP 3 798 063 A1**
(43) Veröffentlichungstag der Anmeldung: **31.03.2021**
(21) Anmeldenummer: 20206419.2
(22) Anmeldetag: 27.03.2020
(51) Int. Cl.: B60R 25/25, G01L 1/14, G01N 27/22, G06Q 30/06, A61B 5/117, A61B 5/01

(54) **CARSHARING VERWALTUNGSSYSTEM MITTELS TEMPERATURSENSOR**

(30) Priorität: 29.03.2019 DE 102019108168
(62) Teilanmeldung aus: 20715340.4
(71) Anmelder: B-Horizon GmbH, 93161 Sinzing (DE)
(72) Erfinder: Kabany, Mohammad, 93053 Regensburg (DE)
(74) Vertreter: Peters, Andreas

(57) **Zusammenfassung**

Carsharing Verwaltungssystem zur Verwaltung von Nutzungsobjekten, insbesondere von Fahrzeugen, umfassend zumindest eine an dem Nutzungsobjekt verbaute Detektionsvorrichtung (100) zur Identifikation des jeweils in einer Datenbank hinterlegten Nutzers, umfassendzumindest einen optischen und/oder physikalischen Sensor (1), der den Nutzer sensorisch detektiert und/oder optische Daten gewinnt sowie zumindest eine Verarbeitungseinheit (5), welche dazu eingerichtet und dafür vorgesehen ist Messwerte des Sensors (1) zu speichern, wobei von der Verarbeitungseinheit (5) die von dem Sensor gemessenen Daten an eine zentrale CPU (40) gesendet werden, wobei diese Daten von der Verarbeitungseinheit (5) verarbeitet werden. Erfindungsgemäß vergleicht die zentrale CPU (40) und/oder die Verarbeitungseinheit (5) diese Daten mit in einer Datenbank hinterlegten oder an die Datenbank gesendete entsprechenden Werten, und, vorzugsweise nur, bei Übereinstimmung mit diesen hinterlegten Werten oder entsprechenden Wertebereichen, sendet die zentrale CPU (40) ein Freigabesignal an eine Abgleicheinrichtung (6), welche daraufhin ein Nutzungsobjekt (7) zur Nutzung freischaltet, insbesondere wobei die zentrale CPU (40) abseits von dem Nutzungsobjekt (7) in zumindest einer, insbesondere ortsfesten, Montagestation verbaut ist, sodass die zentrale CPU (40) drahtlos mit der Abgleicheinrichtung (6) kommunizieren kann.

## Beschreibung

Die vorliegende Erfindung betrifft ein Carsharing Verwaltungssystem zur Verwaltung von Nutzungsobjekten, insbesondere von Fahrzeugen, umfassend den Oberbegriff des Patentanspruchs 1.

Das erfindungsgemäße Carsharing Verwaltungssystem umfasst zumindest eine an dem Nutzungsobjekt verbaute Detektionsvorrichtung zur Identifikation des jeweils in einer Datenbank hinterlegten Nutzers, umfassend zumindest einen optischen und/oder physikalischen Sensor, der den Nutzer sensorisch detektiert und/oder optische Daten gewinnt sowie zumindest eine Verarbeitungseinheit, welche dazu eingerichtet und dafür vorgesehen ist Messwerte des Sensors zu speichern, wobei von der Verarbeitungseinheit die von dem Sensor gemessenen Daten an eine zentrale CPU (Central Processing Unit) gesendet werden, wobei diese Daten von der Verarbeitungseinheit verarbeitet werden.

Die an dem Nutzungsobjekt verbaute Detektionsvorrichtung erkennt dabei bevorzugt eine Chipkarte oder dergleichen des Nutzers oder eine von dem Nutzer in einer entsprechenden App oder einem an dem Nutzungsobjekt angebrachten Bedienterminal eingegebenen Pin. Stimmen das Signal der Chipkarte bzw. der eingegebenen Pin mit den in der Datenbank hinterlegten Werten überein, kann der Nutzer das Nutzungsobjekt, wie insbesondere das Fahrzeug öffnen und nutzen.

Erfindungsgemäß vergleicht die zentrale CPU und/oder die Verarbeitungseinheit diese Daten mit in einer Datenbank hinterlegten Werten oder an die Datenbank gesendete entsprechenden Werten, und, vorzugsweise nur, bei Übereinstimmung mit diesen hinterlegten Werten oder entsprechenden Wertebereichen, sendet die zentrale CPU ein Freigabesignal an eine Abgleicheinrichtung, welche daraufhin ein Nutzungsobjekt zur Nutzung freigibt. Die zentrale CPU ist dabei erfindungsgemäß abseits von dem Nutzungsobjekt in zumindest einer, insbesondere ortsfesten, Montagstation verbaut, so dass die zentrale CPU drahtlos mit der Abgleicheinrichtung kommunizieren kann.

Gemäß zumindest einer Ausführungsform weist das Verwaltungssystem zumindest einen Temperatursensor auf, wobei der Temperatursensor eine Umgebungstemperatur und/oder eine Temperatur eines Sensors und/oder eine Temperatur eine Benutzers des Nutzungsobjekts misst und diesen Wert an die zentrale CPU weiterleitet, insbesondere wobei auf Basis des gemessenen Temperaturwertes des Benutzer eindeutig auf die Identität des Benutzers geschlossen werden kann.

Gemäß zumindest einer Ausführungsform sind die Benutzerdaten, wie Größe, Gewicht und/oder Alter des Benutzers des Nutzungsobjektes, in der zentralen CPU hinterlegt.

Gemäß zumindest einer Ausführungsform ist der Temperatursensor innerhalb des Nutzungsobjektes, vorzugsweise in einer Fahrzeugkanzel verbaut und von dort aus die Temperatur, insbesondere, durch eine Infrarot Wärmemessung, misst.

Gemäß zumindest einer Ausführungsform handelt es sich bei dem Temperatursensor um den Sensor oder aber die beiden Sensoren sind verschieden voneinander.

Mit anderen Worten geht es in vorliegender Erfindung unter anderem darum, dass nur berechtige und/oder geeignete Benutzer das Nutzungsobjekt benutzen können. Bei dem Nutzungsobjekt kann es sich um einen Fahrzeugsitz oder um ein Fahrzeuglenkrad oder einen sonstigen freizuschaltenden Gegenstand handeln. Besonders bevorzugt handelt es sich bei dem Nutzungsobjekt um ein Fahrzeug.

Insbesondere kann es sich daher bei dem hier vorgeschlagenen System um ein System zur Erhöhung der Sicherheit insbesondere im Automotive Bereich handeln.

Die Datenbank kann auf der Abgleicheinrichtung gespeichert sein. Bei einer bevorzugten Ausführungsform ist die Abgleicheinrichtung an dem Nutzungsobjekt verbaut und bewegt sich damit mit dem Nutzungsobjekt mit.

Die Datenkommunikation zwischen der zentralen CPU und/oder der Verarbeitungseinheit und der Abgleicheinrichtung kann drahtfrei, also zum Beispiel über WLAN, Bluetooth oder ähnliches durchgeführt werden.

Bevorzugt sind Die CPU und die Verarbeitungseinheit verschieden voneinander und insbesondere voneinander beabstandet angeordnet.

Gemäß zumindest einer Ausführungsform handelt es sich bei dem Sensor um einen Infrarot, Ultraschall-, UV,- Sensor, einen Laser, einen biometrischen Sensor, Drucksensor, Feuchtigkeitssensor, Temperatur und/oder um eine optische Bildkamera und/oder um einen VCSEL.

Bei einem VCSEL handelt es sich um eine Laserdiode, bei der das Licht senkrecht zur Ebene des Halbleiterchips abgestrahlt wird, im Gegensatz zur kantenemittierenden Laserdiode, bei der das Licht an einer oder zwei Flanken des Chips austritt
Bei einer weiteren bevorzugten Ausführungsform handelt es sich bei dem Sensor um einen Sensor zur Messung von Druck und/oder Feuchtigkeit, wobei der Sensor zumindest einen Kondensator mit zumindest zwei Elektroden umfasst, welche, insbesondere in einer horizontalen Richtung, entlang eines und auf einem, insbesondere flexiblen, Trägermaterial zueinander angeordnet sind, wobei zwischen den Elektroden zumindest eine dielektrische Schicht angeordnet ist, wobei auf einer dem Trägermaterial abgewandten Seite zumindest eine Elektrode und/oder der dielektrischen Schicht zumindest stellenweise, zumindest eine, zumindest teilweise flüssigkeitsdurchlässige und/oder flüssigkeitsabsorbierende Feuchteschicht angeordnet ist, wobei somit die zumindest eine Elektrode und/oder dielektrische Schicht in einer Querrichtung zwischen dem Trägermaterial und der Feuchteschicht angeordnet sind, so dass sich eine Kapazität durch die auf die dielektrische Schicht zumindest teilweise treffende Flüssigkeit, zumindest teilweise verändert, wobei die Verarbeitungseinheit dazu eingerichtet und dafür vorgesehen ist, Messwerte des Sensors zu speichern, so dass ein kapazitiver Feuchtesensor entsteht, wobei von der Verarbeitungseinheit die von dem Sensor gemessenen Daten an die zentrale CPU gesendet werden, wobei diese Daten von der Verarbeitungseinheit verarbeitet werden.

Auch bei der Verwendung eines Sensors zur Messung von Druck und/oder Feuchtigkeit sind die CPU und die Verarbeitungseinheit vorzugsweise verschieden voneinander. Die CPU und die Verarbeitungseinheit sind zum Beispiel voneinander beabstandet angeordnet. Insbesondere können dabei hier die Verarbeitungseinheit und die CPU nicht auf einem gemeinsamen Träger und/oder Substrat angeordnet sein, sofern der Träger nicht das Trägermaterial, also zum Beispiel ein Textil, ist.

Die horizontale Richtung ist vorzugsweise eine Haupterstreckungsrichtung des flexiblen Trägermaterials.

"Flexibel" heißt in diesem Zusammenhang, dass das Trägermaterial zumindest stellenweise biegsam und damit elastisch ist.

Insbesondere kann es sich bei dem Trägermaterial um einen Webstoff oder um einen sonstigen Bekleidungsstoff wie zum Beispiel ein Polyester handeln.

Die dielektrische Schicht beabstandet damit die beiden Elektroden in einer horizontalen und/oder in einer dazu senkrechten Querrichtung.

Ein kapazitiver Feuchtesensor ist im Prinzip ein Kondensator, dessen Dielektrikum vorzugsweise aus einer hygroskopischen Polymerschicht besteht, die entsprechend der Feuchtigkeit der Umgebungsluft Feuchtigkeit aufnimmt (absorbiert) oder abgibt (desorbiert) bis ein Gleichgewichtszustand (Diffusionsgefälle ist = 0) erreicht ist. Dabei verändert sich die Dielektrizitätskonstante des Polymermaterials als Funktion eines Feuchtegehalts.

Die Aufgabe der Verarbeitungseinheit besteht unter anderem darin, vorzugsweise auch aus einer gemessenen Umgebungstemperatur und dem feuchtigkeitsabhängigen Kapazitätswert des Sensors die relative Feuchte möglichst genau zu ermitteln.

Besonders bevorzugt ist der Feuchtesensor in einem Fahrzeugsitz eines Nutzungsobjekts wie insbesondere einem Fahrzeug angeordnet.

Auch ist denkbar, dass die hier beanspruchte Vorrichtung, und insbesondere die Sensoren, auf einer Innenfläche eines Reifen verbaut sind. Auch ist denkbar, dass die Sensoren sogar mit in das Material des Reifen eingefügt sind. Dabei ist vorstellbar, dass die Sensoren alle in das Material eingefügt und damit von dem Material des Reifens ummantelt sind und die Verarbeitungseinheiten auf der Innenfläche des Reifens angeordnet sind. Alternativ können jedoch auch die Verarbeitungseinheiten in das Material des Reifens eingefügt sein. Die Sensoren können dann den Reifeninnendruck, die Reifeninnentemperatur und/oder die Einzel- oder Gesamtlaufzeit des Reifens erfassen.

Gemäß zumindest einer Ausführungsform umfasst das Messsystem zumindest eine Vorrichtung zur Messung von Druck und/oder Feuchtigkeit, wobei die Vorrichtung zumindest einen Sensor zur Messung von Druck und/oder Feuchtigkeit aufweist, wobei der Sensor zumindest einen Kondensator mit zumindest zwei Elektroden umfasst, welche insbesondere in einer horizontalen Richtung entlang eines und auf einem insbesondere flexiblen Trägermaterial zueinander angeordnet sind, wobei zwischen den Elektroden zumindest eine dielektrische Schicht angeordnet ist.

Erfindungsgemäß ist auf eine dem Trägermaterial abgewandten Seite zumindest eine Elektrode und/oder dielektrische Schicht zumindest stellenweise, zumindest eine, zumindest teilweise flüssigkeitsdurchlässige und/oder flüssigkeitsabsorbierende Schicht (= Feuchteschicht) angeordnet, wobei somit die zumindest eine Elektrode und/oder dielektrische Schicht in Querrichtung zwischen dem Trägermaterial und der Feuchteschicht angeordnet sind, sodass eine Kapazität durch die auf die dielektrische Schicht zumindest teilweise treffende Flüssigkeit, zumindest teilweise verändert wird, wobei eine Verarbeitungseinheit dazu eingerichtet und dafür vorgesehen ist diese Messwerte des Sensors zu messen und/oder zu speichern, sodass ein kapazitiver Feuchtesensor entsteht.

Erfindungsgemäß werden von der Verarbeitungseinheit die von dem Sensor gemessenen Daten an eine zentrale CPU (Central Processing Unit) gesendet, wobei diese Daten von der Verarbeitungseinheit verarbeitet werden.

Insbesondere kann in der Datenbank ein, beispielsweise genau ein, Feuchtigkeitsgrenzwert und/oder Druckgrenzwert hinterlegt sein, bis zu dem eine Freischaltung des Nutzungsobjekts erfolgt.

Gemäß zumindest einer Ausführungsform umfasst das Carsharing Verwaltungssystem zumindest eine Sendeeinrichtung, welche an die Abgleicheinrichtung vorgegebene Werte oder Wertebereiche der Druck- und/oder Feuchtigkeitswerte und/oder Temperaturwerte und/oder optischen gewonnen Werte sendet, und die Abgleicheinrichtung diese empfangenen Werte mit den von der zentralen CPU an die Abgleicheinrichtung gesendeten Werte oder Wertebereiche vergleicht.

Gemäß zumindest einer Ausführungsform handelt es sich der Sendeeinrichtung um ein Fahrzeugzugangssystem, zum Beispiel um einen Fahrzeugschlüssel, oder diese ist zumindest ein Teil davon, wobei die Sendeeinrichtung dazu eingerichtet und dafür vorgesehen ist mittels einer drahtlosen Verbindung mit der Abgleicheinrichtung und/oder der zentralen CPU zu kommunizieren. Der Fahrzeugschlüssel kann in einem Mobilgerät (Handy), Hardware und/oder Software integriert sein.

Zum Beispiel kann so eine Schlüsselerkennung realisiert sein. Insbesondere kann ein Schlüssel nämlich einem oder mehreren, vorzugsweise genau einem, Benutzer zugeordnet sein. Jede Sendeeinrichtung, insbesondere damit auch jeder Schlüssel, kann einem ganz bestimmter Druck- und/oder Feuchtewert und/oder physikalischen und/oder optischen Wert welcher in der Datenbank hinterlegt ist, entsprechen. Fällt der durch die Vorrichtung gemessene Druck-und/oder Feuchtewert (wenn ein Benutzer zum Beispiel auf einem Fahrzeugsitz Platz nimmt) nicht in einen Normnutzungsbereich (Bereich von Druck- und/oder Feuchtewert innerhalb dessen eine Benutzung erlaubt wird) so kann eine Nutzung des Fahrzeugsitzes insbesondere jedoch des gesamten Fahrzeugs versagt werden. Zum Beispiel bleib die Zündung des Fahrzeugs aus oder das Lenkrad versperrt. Gleiches gilt bevorzugt auch, wenn ein Signal beispielsweise einer Chipkarte des Nutzers nicht mit den in der Datenbank hinterlegen Werten des Nutzers übereinstimmt oder durch den Nutzer ein falscher Pin in einer App oder einem Bedienterminal an dem Nutzungsobjekt eingegeben wird. Hier kann zum Beispiel das Fahrzeug erst gar nicht geöffnet werden.

Werden optische Daten erfasst, wird beispielsweise mit einer Bildkamera eine Gesichtserkennung vorgenommen oder über einen Fingerabdrucksensor ein Fingerabdruck des Nutzers überprüft. Auch hier wird dann, falls diese Daten nicht mit den in der Datenbank hinterlegten Daten des Nutzers übereinstimmen, beispielsweise durch nicht öffnen des Fahrzeugs, Einsetzen einer Lenkradsperre oder dergleichen verhindert, dass der Nutzer das Objekt nutzt.

Auch ist denkbar, dass von einem Benutzer erzeugte Druck- und/oder Feuchtewerte und/oder physikalische und/oder optische Werte von der Vorrichtung gemessen werden und dann in den Schlüssel eingespeist werden. Hierzu kann der Schlüssel einen Speicherchip aufweisen.

Wird der Schlüssel nun wiederbenutzt kann sich aufgrund der gespeicherten Druck- und/oder Feuchtewerte beispielsweise vor Belegung des Fahrzeugsitzes der Sitz automatisch in seiner Form und Sitzposition an den Benutzer anpassen.

Mit Hilfe der gemessenen Druck- und/oder Feuchtigkeitswerten kann mit dem hier vorgeschlagenen System zusätzlich zur Erhöhung der Sicherheit auch der Komfort im Automotive Bereich erhöht werden.

Denkbar ist auch, dass nach einer Sitzbelegung des Fahrzeugsitzes abhängig von der identifizierten Person eine vorgespeicherte Sitzposition, ein vorgespeichertes Licht- und /oder Schallambiente, ein vorgespeicherter Musik- und/oder Radiosender oder andere personalisierte Dienste ausgewählt und/oder angewendet werden. Diese Einstellungen können dabei auch aufgrund der Gesichtserkennung oder der Erkennung der Chipkarte des Nutzers oder dergleichen automatisch eingerichtet werden.

Gemäß zumindest einer Ausführungsform ist die Abgleicheinrichtung in den Fahrzeugschlüssel integriert verbaut. Alternativ oder zusätzlich kann die Abgleicheinrichtung auch in die zentrale CPU und/oder die Verarbeitungseinheit verbaut sein. Zudem ist es möglich, dass die zentrale CPU und/oder die Verarbeitungseinheit die Rolle der Abgleicheinrichtung übernehmen. In diesem Fall kann auf eine separate Abgleicheinrichtung verzichtet sein.

Gemäß zumindest einer Ausführungsform gibt die Abgleicheinrichtung ein Fahrzeugschloss, eine Wegfahrsperre, ein Lenkradschloss, ein Gas- und/oder Bremspedal zur Nutzung bei einer Datenübereinstimmung frei. Bei diesen Elementen kann es sich jeweils um ein Nutzungselement handeln.

Gemäß zumindest einer Ausführungsform wird auf Basis der einzelnen Werte die zentrale CPU zumindest einen jeweiligen Kennwert ermittelt, aus welchem ableitbar ist, welches Individuum nach Gewicht und/oder Größe und/oder Körperabdruck und/oder sonstigen physikalischen Eigenschaften gerade die Vorrichtung zur Messung von Druck und/oder Feuchtigkeit besetzt, und/oder dass nach einer Sitzbelegung des Fahrzeugsitzes abhängig von der identifizierten Person eine vorgespeicherte Sitzposition, ein vorgespeichertes Licht- und /oder Schallambiente und/oder ein Klima, ein vorgespeicherter Musik- und/oder Radiosender oder andere personalisierte Dienste ausgewählt und/oder angewendet werden.

Im Sinne der Erfindung handelt es sich bei einem Körperabdruck um eine räumliche und/oder flächige Ausdehnung eines Körperumrisses auf einem Nutzungsobjekt, wie einem Fahrzeugsitz. Der Körperumriss kann durch ein vom Körper auf dem Bezugsstoff generiertes Temperatur- und/oder Druckfeld begrenzt und definiert sein. Dieser Körperabdruck kann einzigartig sein und genau einem Benutzer zugeordnet werden. Ein solcher Körperabdruck kann daher auch als ein biometrisches Muster, insbesondere als ein biometrischer Körperabdruck, bezeichnet werden. Die von einer Messmatrix der hier beschriebenen Vorrichtung (es können die hier beschriebene Sensoren matrixartig unterhalb und entlang der Fahrzeugsitzoberfläche angeordnet sein) gemessenen Temperatur und/oder Feuchtigkeits- und/oder Gewichtswerte können so zu einem gemeinsamen Messbild vereint werden, was in der Summe damit den Körperabdruck eines einzigen Benutzer ergeben kann.
Gemäß zumindest einer Ausführungsform gilt, dass sofern die gemessenen Daten mit den hinterlegten Daten nicht übereinstimmen und/oder um mehr als 25% von den hinterlegten Datenwerten abweichen eine Gesichtserfassungseinrichtung und/oder Körperabdruckerfassungseinrichtung ein Gesicht und/oder einen Körperabdruck des Benutzers auf Basis von Bilddaten, welche den optischen gewonnenen Daten entsprechen oder aus diesen gewonnen sind, erfasst, und trotz der Datenabweichung dann das Nutzungselement freischaltet, sofern die Gesichtserfassungseinrichtung ein Benutzergesicht als identisch mit einem in der Gesichtserfassungseinrichtung hinterlegten Gesicht identifiziert.

Gemäß zumindest einer Ausführungsform werden die erfassten Bilddaten mit den Berechtigungsdaten verglichen, die ebenso in der Datenbank hinterlegt sind, wobei dieser Vergleich einen Klassifikationsvergleich von Datenklassen der Bilddaten mit Datenklassen der Berechtigungsdaten umfasst, wobei eine Klassifikation der Bilddaten auf Basis von Bewegungsvektoren des Benutzers derart durchgeführt wird, dass zunächst aus der zeitlichen Bewegung des Benutzers ein Bewegungsprofil von der Gesichtserfassungseinrichtung erstellt wird, wobei das Bewegungsprofil des Benutzers Bewegungsvektoren desselbigen umfasst, und weiter wobei ein Benutzer und/oder Benutzerbewegungen zu sogenannten Datenblops oder Datenclustern zusammengefasst werden, die dann in ihrer Form und/oder Ausdehnung klassifiziert werden.

Der Sensor und/oder die Verarbeitungseinheit und/oder die zentrale CPU können mittels einer Batterie oder einer Festnetzstromversorgung mit elektrischer Energie versorgt werden.

Alternativ oder zusätzlich ist die Erzeugung von elektrischer Energie zur Versorgung des Sensors und/oder Verarbeitungseinheit mittels sogenannten "Energy Harvesting" möglich.

Als Energy Harvesting (wörtlich übersetzt *Energie-Ernten*) bezeichnet man die Gewinnung kleiner Mengen von elektrischer Energie aus Quellen wie Umgebungstemperatur, Vibrationen oder Luftströmungen für mobile Geräte mit geringer Leistung. Die dafür eingesetzten Strukturen werden auch als Nanogenerator bezeichnet. Energy Harvesting vermeidet bei Drahtlostechnologien Einschränkungen durch kabelgebundene Stromversorgung oder Batterien.

Möglichkeiten des Energy Harvesting:
- Piezoelektrische Kristalle erzeugen bei Krafteinwirkung, beispielsweise durch Druck oder Vibration, elektrische Spannungen. Diese Kristalle können an oder auf dem Trägermaterial angeordnet sein.
- Thermoelektrische Generatoren und pyroelektrische Kristalle gewinnen aus Temperaturunterschieden elektrische Energie. Diese Generatoren können an oder auf dem Trägermaterial angeordnet sein.
- Über Antennen kann die Energie von Radiowellen, eine Form von elektromagnetischer Strahlung, aufgefangen und energetisch verwendet werden. Ein Beispiel dafür sind die passiven RFIDs. Diese Antennen können an oder auf dem Trägermaterial angeordnet sein.
- Photovoltaik, elektrische Energie aus der Umgebungsbeleuchtung.
- Osmose.

Ein Energiespeicher der Vorrichtung kann Teil einer Verarbeitungseinheit sein. Hierzu können eine oder mehrere der Verarbeitungseinheiten einen solchen Energiespeicher (lokale Energiespeicher) aufweisen. Zum Beispiel weisen nur eine oder einige der Verarbeitungseinheiten einen solchen Energiespeicher auf, sodass eine dieser Verarbeitungseinheiten eine andere Verarbeitungseinheit (nämlich eine solche, welche keinen Energiespeicher aufweist) mit elektrischer Energie versorgt.

Auch ist denkbar, dass die Energiespeichereinheit(en) der Verarbeitungseinheit(en) die CPU mit elektrischer Energie ganz oder teilweise versorgt. Zum Beispiel kann so die CPU an keine weiteren Energiespeicher und/oder Energieversorgungsleitungen angeschlossen sein.

Über das oben genannte Energy Harvesting kann zumindest einer der Energiespeicher geladen werden.

Die Energieübertragung zwischen den Sensoren und/oder den Verarbeitungseinheiten und/oder der CPU kann ganz oder teilweise drahtlos erfolgen.

Zu der drahtlosen Energieübertragungen im Nahfeld, auch als nicht strahlende Kopplung bezeichnet, zählt beispielsweise die induktive Kopplung, basierend auf dem magnetischen Fluss. Häufig wird die Bezeichnung der drahtlosen Energieübertragung synonym für die induktive Energieübertragung verwendet, da diese in praktischen Anwendungen eine dominante Rolle einnimmt. Bei der nicht strahlenden Kopplung im Nahfeld spielen Wellenphänomene keine Rolle.

Beispielsweise geschieht die drahtlose Energieübertragung zwischen den einzelnen Elementen mittels induktiver Kopplung, resonanter induktiver Kopplung und/oder kapazitiver Kopplung.

Gemäß zumindest einer Ausführungsform weist das Kontrollsystem zumindest zwei Sensoren auf, wobei durch die Verarbeitungseinheit die Sensoren in Gruppen aus zumindest einem Sensor auf Basis zumindest einem der folgenden Kriterien unterteilt werden:
- Anordnungsort des Sensors oder der Sensoren auf dem Trägermaterial, wobei das Trägermaterial in Flächengebiete eingeteilt ist, und innerhalb
- eines Flächengebiets nur Sensoren einer Gruppe angeordnet sind,
- Flächenausdehnung eines Sensors.

Gemäß zumindest einer Ausführungsform umfasst das Kontrollsystem zumindest zwei Vorrichtungen zur Messung von Druck und/oder Feuchtigkeit und/oder zur Messung von physikalischen und/oder optischen Daten, wobei jede Verarbeitungseinheit ihre von den Sensoren empfangenen Daten an die zentrale CPU weiterleitet. Die Datenverbindung zwischen der Verarbeitungseinheit und der zentralen CPU kann kabelgebunden (mit Datenverbindungen) oder aber Wireless erfolgen. Hierzu kann zumindest eine Verarbeitungseinheit eine Bluetooth Verbindung zu der zentralen CPU aufbauen.

Gemäß zumindest einer Ausführungsform umfasst zumindest eine Vorrichtung zumindest zwei Sensoren. Insofern kann eine Sensorgruppe bereits durch diese zwei Sensoren gebildet sein. Die beiden Sensoren könne dann durch eine gemeinsame Verarbeitungseinheit gesteuert und/oder geregelt werden.

Denkbar ist, dass die Vielzahl von Verarbeitungseinheiten ein Verarbeitungsnetzwerk ausbilden, wobei die Erfassung, Verarbeitung und/oder Weitergabe der Sensordaten und/oder der Verarbeitungsdaten eines jeden Sensors und/oder einer jeden Verarbeitungseinheit durch zumindest eine Steuerungseinrichtung (Master) gesteuert wird. Die Steuerungseinheit kann identisch mit der oben beschriebenen CPU sein.

Es ist jedoch auch möglich, dass eine oder mehrere der Verarbeitungseinheiten den Master darstellen, welcher die übrigen Verarbeitungseinheiten (Slave) und/oder die übrigen Sensoren (Slave) steuern.

Zum Beispiel kann eine der Verarbeitungseinheiten und/oder die CPU nach Inbetriebnahme der Vorrichtung (zum Beispiel nach einem Anschalten der Vorrichtung), solche Sensoren auswählen, welche für eine vorgebbare Nutzungsdauer in Betrieb genommen werden. Alternativ können auch alle oder einige Sensoren in Betrieb genommen werden, dann jedoch ist vorstellbar, dass eine Verarbeitungseinheit und/oder die CPU, insbesondere zum Zwecke der Energieersparnis, nur Daten einer vorgegebenen Anzahl (also weniger als alle Sensoren) von Sensoren an die CPU weiterleitet (Filterung).

Diese Master-Verarbeitungseinheit kann vorzugsweise als einzige Einheit mit der CPU kommunizieren.

Weiter alternativ oder zusätzlich ist vorstellbar, dass eine oder alle Verarbeitungseinheiten und/oder ein Sensor (Slave oder Master) direkt mit der CPU kommunizieren.

Gemäß zumindest einer Ausführungsform ist das Verarbeitungsnetzwerk mittels zumindest eines VLAN-Switches in zumindest zwei, nur logisch voneinander getrennte, Netzwerksegmente (VLANs) unterteilbar und wobei jedes der Erfassungselemente in Abhängigkeit von der Ansteuerung durch einen VLAN-Switch und/oder der Steuerungseinrichtung und somit durch jedes der Netzwerksegmente ansteuerbar ist.

Wird zum Beispiel eine sehr große Fläche (zum Beispiel ein Textil) mit einer Vielzahl von hier beanspruchten Sensoren und Verarbeitungseinheiten bestückt können in besonders einfacher Art und Weise dann einzelne Verarbeitungseinheiten und/oder Sensoren kategorisiert werden (nach verschiedenen Prioritäten etc.). Es wird somit in einer Ausführungsform statt einer physikalischen Netzwerkunterteilung eine "virtuelle", das heißt VLAN-Unterteilung gewählt. Dies gewährleistet nämlich, dass auf Veränderungen in der Kategorisierung der Verarbeitungseinheiten und/oder Sensoren besonders schnell und ohne aufwendige Umbauarbeiten reagiert werden kann.

Gemäß zumindest einer Ausführungsform umfasst das Kontrollsystem zumindest ein Verarbeitungsnetzwerk, wobei mittels zumindest eines VLAN-Switches des Verarbeitungsnetzwerks, dieses in zumindest zwei, nur logisch voneinander getrennte, Netzwerksegmente (VLAN) unterteilbar ist, und wobei jeder Verarbeitungseinheiten und/oder jeder der Sensoren in Abhängigkeit von der Ansteuerung durch den VLAN-Switch durch jedes der Netzwerksegmente ansteuerbar sind.

Dazu kann der VLAN-Switch in zumindest einem der Verarbeitungseinheiten und/oder Sensoren oder in einem separaten Bauteil verbaut sein.

Gemäß zumindest einer Ausführungsform wird mittels des VLAN-Switches eine Priorisierung der einzelnen Netzwerksegmente insbesondere im Hinblick auf deren Datenaustausch durchgeführt.

Gemäß zumindest einer Ausführungsform ist jeder Verarbeitungseinheit und/oder jedem Netzwerksegment zumindest eine VLAN-ID zugeordnet, wobei über jede der VLAN-IDs zumindest ein Sensor oder eine andere Verarbeitungseinheit ansteuerbar ist. Einzelne Sensoren und/oder einzelne Verarbeitungseinheiten können ein eigenes Sub-Netzwerk bilden.

Um über Netzwerkgrenzen zu kommunizieren, werden im Stand der Technik statische projektdynamische Routen eingesetzt. Dieses Modell der Trennung ist klar und übersichtlich und wurde über Jahre angewandt. Dies hat jedoch die Nachteile, dass Broadcastanforderungen im Subnetz für alle Teilnehmer sichtbar sind und von den Endpunkten betrachtet werden müssten. Mit anderen Worten, konnten bisher verschiedene Endgeräte nur über entsprechende jedem Subnetz zugeordnete, separate und physikalisch voneinander getrennte Switches angesteuert werden. Ein derartiger Aufbau ist jedoch besonders kostspielig und umfangreich im Design.

Wie obig bereits erwähnt, ist somit insbesondere auf die Ausgestaltung eines jeden Subnetzwerks mit einem separaten Switch und separaten physikalischen Datenleitungen verzichtet, sodass für das gesamte Netzwerk eine einzige physikalische Struktur angewandt werden kann, wobei diese physikalische Struktur, d. h. Netzwerkarchitektur, nur aufgrund einer logischen, insbesondere auch mathematischen Unterscheidung (d. h. gedacht, aufgetrennt wird.

Dabei bezeichnet die Abkürzung "VLAN-Switch" einen solchen Netzwerkswitch, welcher dazu eingerichtet und dafür vorgesehen ist, ein Netzwerk in Form eines Virtual Local Area Network (VLAN) mit zu betreiben.

Insofern wird somit durch die nunmehr beanspruchten Netzwerksegmente, welche jeweils in Form eines VLAN-Netzwerks ausgebildet sein können, es ermöglicht, dass die Trennung des Netzwerks in mehrere logische Segmente, also die Netzwerksegmente, unterteilt wird.

Anders als bei der physikalischen Trennung durch die Zuordnung zu einem Switch Port werden bei der Trennung durch VLANs die Geräte durch eine VLAN-ID logisch getrennt. Dabei wird der Datenstrom jeder Station mit einer Kennung (dem VLAN-"Tag") versehen. Diese Kennung bestimmt die Zugehörigkeit eines Datenpakets zu einem bestimmten VLAN. Alle Geräte mit der gleichen VLAN-Kennung befinden sich nunmehr in einem logischen Netzwerk.

Insbesondere kann nämlich durch die logische Trennung der einzelnen Netzwerke ein Broadcast eingegrenzt werden. Broadcasts werden nämlich nur an Mitglieder des gleichen VLANS und nicht an alle am Switch hängenden Steuerelemente verteilt.

Dies trägt insofern auch nicht nur zu einer höheren Leistung, sondern auch zu mehr Sicherheit bei, denn der Datenverkehr wird auf weniger Adressaten eingeschränkt. Hinzu kommt, dass Benutzer oder die Steuerelemente in der Regel in einem VLAN keine Möglichkeit haben aus dem zugewiesenen VLAN auszubrechen. Ein Zugriff (oder Angriff) auf einen anderen Rechner, der nicht zum eigenen VLAN gehört kann daher bereits durch den Netzwerkswitch verhindert werden. Wenn eine VLAN übergreifende Kommunikation notwendig ist, können hierfür explizit Routen eingerichtet werden.

Insbesondere wird darauf hingewiesen, dass die hier beschriebene VLAN-Technologie eine solche sein kann, welche an den Industriestandard IEEE 802.1Q angepasst ist und/oder mit diesem kompatibel ist.

Der Standard IEEE 802.1Q ist eine durch das IEEE genormte Priorisierungs- und VLAN-Technologie, die im Unterschied zu den älteren, nur Port-basierten VLANs, paketbasierte tagged-VLANS implementiert. Der Ausdruck "tagged" leitet sich vom englischen Ausdruck "material tags" ab.

Es handelt sich daher bei tagged-VLANS um Netze, die Netzwerkpakete verwenden, welche eine spezielle VLAN-Markierung tragen.

Insbesondere sind nämlich in den 802.1Q-Standard Datenfelder für das V-LAN-tagging definiert, die in dem Datenbereich eines Ethernetpakets eingeführt werden können.

Insofern kann das vorliegende Netzwerk in Form eines Ethernet-Kommunikationssystems ausgebildet sein.

Das hat den Vorteil, dass in der Regel auch bereits vorhandene, ältere Switches solche Pakete weiterleiten können. Das eingefügte Tag besteht in der Regel aus mehreren Feldern, beispielsweise vier Feldern mit einer Gesamtlänge von 32 Bit.

Für die Protokoll-ID werden 2 Byte, für das Prioritätenfeld 3 Bit, für Indikator des Canonicalformats 1 Bit und für VLAN-ID 12 Bit genutzt.

Zur eindeutigen Identifizierung eines VLANS wird jedem VLAN daher zunächst eine eindeutige Nummer zugeordnet. Man nennt diese Nummer VLAN-ID. Ein Erkennungsmodul, das mit der VLAN-ID = 1 ausgestattet ist, kann mit jedem anderen Gerät im gleichen VLAN kommunizieren, nicht jedoch mit einem Gerät in einem anderen VLAN, wie z. B. ID = 2, 3,....

Um zwischen den VLANS zu unterscheiden, wird nach dem Standard IEEE 802.1 Q ein Ethernetframe um 4 Byte erweitert. Davon sind 12 Bit zur Aufnahme der VLAN-ID vorgesehen, sodass (ohne Ausnutzung des Canonical Formats) theoretisch 4096-2 = 4094 VLANS möglich sind.

Denkbar ist, dass die einzelnen logischen Netzwerkverbindungen entsprechend eines OPC-Standards, d. h. beispielsweise in Form von OPC UA-Verbindungen, ausgebildet sind. Insbesondere ist nämlich denkbar, dass pro Netzwerksegment über die Steuerungseinrichtung mehrere OPC UA-Endpunkte mit unterschiedlicher IP-Adresse, VLAN-ID und Priorisierung entsprechend des oben genannten Standards IEEE 802.1Q zur Verfügung stehen.

Weist nun ein Netzwerksegment, welches eindeutig, vorzugsweise eineindeutig, eine bestimmte VLAN-ID zugewiesen bekommen hat, eine höhere Priorität auf als ein, nur in logischer Hinsicht, davon unterschiedliches Netzwerksegment einer entsprechend unterschiedlichen VLAN-ID, so können die Steuerungseinrichtung und/oder der VLAN-Switch dazu vorgesehen sein, zunächst den Datenaustausch des höher priorisierten Netzwerksegments zu bevorzugen, um erst nach Abbau der diesem höher priorisierten Netzwerksegment zugeordneten Aufgaben eine Abarbeitung des tiefer priorisierten Netzwerksegments zu erlauben.

Mit anderen Worten gilt generell: Zuordnung und Konfiguration der OPC UA Endpunkte zu einem bestimmten Netzwerksegment entsprechend der VLAN-ID und Zuordnung einer Priorität entsprechend der Priorität des entsprechenden VLANs.

Gemäß zumindest einer Ausführungsform sind jedem Sensor und/oder jeder Verarbeitungseinheit zumindest eine VLAN-ID zugeordnet und jedem Netzwerksegment ist wiederum zumindest eine, beispielsweise genau eine, eindeutig, vorzugsweise eineindeutig, VLAN-ID zugeordnet, wobei über jede der VLAN-IDs zumindest ein Steuerelement ansteuerbar ist. Gemäß zumindest einer Ausführungsform umfasst zumindest eine Vorrichtung zumindest einen Temperatursensor, wobei der Temperatursensor eine Umgebungstemperatur und/oder eine Temperatur eines Sensors misst und an die Verarbeitungseinheit einer Vorrichtung und/oder an die zentrale CPU weiterleitet.

Gemäß zumindest einer Ausführungsform ermittelt die zentrale CPU einen Auslastungsgrad (CPU-Last und/oder Speicherverbrauch) von zumindest einer Verarbeitungseinheit, wobei bei Überschreiten einer Grenztemperatur der Verarbeitungseinheit und/oder zumindest der dieser Verarbeitungseinheit zugeordneten Sensor diese(r) in seiner Leistung zumindest teilweise gedrosselt oder ganz abgeschaltet wird

Gemäß zumindest einer Ausführungsform ist der Sensor zusätzlich ein kapazitiver Drucksensor, wobei die Verarbeitungseinheit zusätzlich dazu eingerichtet und dafür vorgesehen ist, eine durch äußeren Druck verursachte Kapazitätsänderung des Kondensators zu messen und/oder zu speichern.

Grundsätzlich handelt es sich bei einem kapazitiven Sensor also um einen Sensor, welcher auf Basis der Veränderung der elektrischen Kapazität eines einzelnen Kondensators oder eines Kondensatorsystems arbeitet. Die Beeinflussung der Kapazität durch die zu erfassende Größe kann dabei auf verschiedene Arten, erfolgen die primär durch den Verwendungszweck bestimmt ist.

Ein kapazitiver Sensor basiert unter anderem darauf, dass zwei Elektroden, einer davon kann die zumessende Oberfläche sein, die "Platten" eines elektrischen Kondensators bilden, dessen Kapazität oder Kapazitätsänderung gemessen wird, die folgendermaßen beeinflusst werden kann:
- Eine Platte wird durch den zumessenden Effekt verschoben und/oder verformt, wodurch sich der Plattenabstand und damit die elektrische messbare Kapazität ändern.
- Die Platten sind starr und die Kapazität an Sich ändert sich dadurch, dass ein elektrisch leitendes Material oder ein Dielektrikum in unmittelbare Nähe gebracht wird.
- Die wirksame Plattenfläche ändert sich, indem die Platten wie bei einem Drehkondensator gegeneinander verschoben werden.

Um auch kleine Veränderungen besser detektieren zu können kann die eigentliche Messelektrode häufig mit einer Schirmelektrode umgeben sein, die den inhomogenen Randbereich des elektrischen Feldes von der Messelektrode abschirmt, dadurch ergibt sich zwischen Messelektroden üblicherweise geerdeter Gegenelektrode eine annähernd paralleles elektrisches Feld mit der bekannten Charakteristik eines idealen Plattenkondensators.

Ein kapazitiver Drucksensor ist insbesondere ein solcher, bei dem die Kapazitätsänderung infolge des Durchbiegens einer Membran und der resultierenden Änderung des Plattenabstands als Sensoreffekt ausgewertet wird. Zum Beispiel handelt es sich bei der Membran um das oben genannte Dielektrikum oder aber um die einzelnen Kondensatorelektroden welche insbesondere in Form einer Platte ausgeführt sein können. Mit anderen Worten ist in einer derartigen Ausführungsform in neuartiger Art und Weise ein kapazitiver Feuchtesensor mit einem kapazitiven Drucksensor kombiniert, jedoch ohne dass diese Bauteile voneinander getrennte Elemente oder zwei separate Sensoren bildeten, sondern es handelt sich bei vorliegender Ausführungsform um ein "Two in One"-Konzept, in welchem der gleiche Sensor sowohl als Feuchtesensor, als auch als Drucksensor fungiert.

Gemäß zumindest einer Ausführungsform handelt es sich bei dem Trägermaterial um einen Webstoff, insbesondere in welchem elektrische Leiterbahnen zur elektrischen Kontaktierung des Sensors und der Verarbeitungseinheit eingewoben sind.

Bei einem Webstoff handelt es sich im Sinne der Erfindung daher um ein Gewebe, welches manuell oder maschinell auf Basis von einzelnen Fäden gewebt wurde.

Die elektrischen Leiterbahnen können in einem Gewebe daher zusätzlich neben den üblichen Fasern und Gewebesträngen integriert sein oder aber einzelne Gewebestränge welche das Gewebenetz ausbilden ersetzen.

Je nach Abstand und Eigenschaften der einzelnen Fäden (hochgedreht, bauschig, usw.) können ganz lockere Gewebe, wie Verbandgewebe oder Dichtegewebe wie Brokatstoff entstehen. Längselastisch werden Gewebe durch, als Kettenfäden eingesetzte Gummifäden (mehr Bändern verwendet) oder Kräusel- und Bauschgarne verwendet. Sie werden gespannt, verarbeitet und ziehen sich im Ruhezustand zusammen. Bauschgarne bestehen aus texturiertem, also gekräuseltem synthetischen Fasern. Die Kräuselung verändert die Eigenschaften der synthetischen Fasern. Die darauf gesponnenen Garne sind sehr elastisch und voluminös und haben eine gute Wärmedämmung.

Zum Beispiel kann das Trägermaterial Teil eines Bezugstoffes eines Sitzes, insbesondere eines Fahrzeugsitzes oder eines Bürostuhls, sein. Insofern kann der Sensor, vorzugsweise jedoch die gesamte Vorrichtung, auf dem Bezugsstoff eines solchen Sitzes aufgebracht oder in einen solchen integriert sein.

Zum Beispiel ist die Verarbeitungseinheit dazu eingerichtet und dafür vorgesehen, die einzelnen Feuchte- sowie Druckwerte zu erfassen und aus einer Kombination der einzelnen Feuchte- und Druckwerte zumindest einen jeweiligen Kennwert zu ermitteln, aus welchem ableitbar ist, welches Individuum (mit Gewicht und/oder Größe) gerade den Fahrzeugsitz besetzt.

Zum Beispiel kann aus der Druckmessung durch die Verarbeitungseinheit ein Gewicht der jeweiligen Person abgeleitet und festgestellt werden. Auch kann die jeweilige Feuchtigkeit, welche die jeweilige Person an den Sensor abgibt, gemessen werden, wobei der jeweilige Kennwert zum Beispiel ein Produkt aus dem relativen Feuchtigkeitswert mal der von der Verarbeitungseinheit ermittelten Belastungsgewicht ist.

Überschreitet ein derartiger Kennwert einen entsprechenden Grenzwert kann die Verarbeitungseinheit insbesondere mittels einer Anbindung an die Elektronik des Fahrzeugs, eine Warnung aussprechen. Diese Warnung kann dahingehend lauten, dass der Sitz überbelegt ist oder der Fahrer zu stark schwitzt. Diese Warnung kann jedoch auch ersetzt werden durch eine entsprechende Anzeige dahingehend welcher Belegungstyp den Sitz nutzt. Bei einem Belegungstyp kann es sich um eine Gewichtsklassifikation eines jeweiligen Benutzers handeln, oder aber auch darum Handeln ob es sich bei dem Benutzer um ein Tier, einen Menschen oder auch um eine Sache handelt. Vorzugsweise ist daher die Verarbeitungseinheit in eine Anzeigenelektronik des Fahrzeugs integrierbar, zumindest jedoch mit einer solchen verbindbar.

Hierzu ist denkbar, dass die Verarbeitungseinheit sich zum Beispiel mittels Bluetooth oder einer sonstigen Wireless Verbindung mit einer Empfangseinheit des Fahrzeugs verbindet und der jeweilige Kenn- oder Grenzwert und/oder die jeweilige Warnung und/oder die jeweilige Identifikation des Benutzers auf einem Display des Fahrzeugs wiedergegeben werden. Eine Identifikation des Benutzers kann dabei jedoch auch über eine Gesichtserkennung, einen Fingerabdruck, ein Signal einer Chipkarte oder einen Pin oder dergleichen erfolgen.

Alternativ oder zusätzlich ist vorstellbar, dass diese einzelnen Werte und/oder Identifikationen auch extern abrufbar und/oder extern darstellbar sind. Zum Beispiel kann das Auto auf eine Überbelegung hin von einem externen Controller überwacht werden.

Zum Beispiel kann mittels einer Datenverbindung die Verarbeitungseinheit mit einer Auslöseinheit eines Airbags in Verbindung stehen, sodass die Verarbeitungseinheit auch die Auslöseinheit steuern und/oder regeln kann, insbesondere in Bezug auf einen Auslösezeitpunkt des Airbags. Zusätzlich und/oder alternativ ist es möglich, dass die Verarbeitungseinheit eine Controllereinheit des Airbags mit Daten zum Beispiel im Hinblick auf einen Belegungstyp, Position und/oder Gewicht eines Benutzers des Fahrzeugsitzes versorgt.

Diese Daten können dazu führen, dass der Auslösezeitpunkt und die Auslösereihenfolge des Airbags auf den Benutzer angepasst sind, sodass ein Personenschaden an dem Benutzer vermieden wird.

Gemäß zumindest einer Ausführungsform ist zumindest eine Elektrode und/oder dielektrische Schicht auf dem Trägermaterial oder auf einer dem Trägermaterial angeordnete, insbesondere wasserundurchlässigen Schicht aufgedruckt oder mittels eines Dünnschichtverfahrens aufgebracht.

Dies heißt, dass zumindest ein Element, vorzugsweise sowohl die Elektrode als auch die dielektrische Schicht, auf dem Trägermaterial oder einer zwischen dem Sensor und dem Trägermaterial aufgebrachten vorzugsweise elektrisch nicht leitfähigen, weiter vorzugsweise wasserundurchlässigen Schicht mittels eines Druckverfahrens aufgedruckt.

Bei dem Druckverfahren kann es sich zum Beispiel um ein Inkjetverfahren handeln.

Zum Beispiel ist die Verarbeitungseinheit in der gleichen Weise wie der Sensor auf das Trägermaterial aufgebracht. Hierzu ist vorstellbar, dass auch die Verarbeitungseinheit, zumindest jedoch eine, insbesondere leitende, Schicht der Verarbeitungseinheit auf das Trägermaterial zum Beispiel aufgedruckt ist. Die Datenkommunikation zwischen der Verarbeitungseinheit und dem Sensor kann dann über die oben genannten Leiterbahnen entstehen. Diese Leiterbahnen können zumindest teilweise, vorzugsweise jedoch vollständig, in den Webstoff eingewoben sein oder sogar einzelne Fasern des Webstoffs selbst ausbilden.

Zum Beispiel ist zumindest eine Elektrode flächig ausgeführt. Das heißt, dass eine Dicke der Elektrode im Vergleich zu deren Flächenausdehnung vernachlässigbar ist. Eine solche Elektrode kann daher insbesondre mittels eines Druckverfahrens hergestellt werden.

Alternativ hierzu kann eine Dicke zumindest einer Elektrode höchstens 5 mm betragen. Hierzu kann das Druckverfahren mehrmals angewandt werden, sodass zumindest zwei, vorzugsweise jedoch dann mehr, Einzeldruckschichten übereinander gestapelt werden.

Des Weiteren kann die Elektrode auch mittels eines 3D-Druckverfahrens auf dem Trägermaterial angeordnet sein.

### 1. Das FDM-Verfahren (Fused Deposition Modeling)

### Alternativbezeichnungen: Fused Filament Fabrication (FFF), Fused Layer Modeling (FLM)

Das Verfahren bezeichnet schichtweises Auftragen (Extrusion) eines Materials durch eine heiße Düse. Das Verbrauchsmaterial befindet sich in Form eines langen Drahts (sog. Filament) auf einer Rolle und wird durch die Fördereinheit in einen Druckkopf geschoben, dort eingeschmolzen und auf einem Druckbett ausgebracht. Druckkopf und/oder Druckbett sind dabei in drei Richtungen beweglich. So können Kunststoffschichten schrittweise aufeinander aufgebracht werden.

### 2. Das SLS Verfahren (Selektives Lasersintern)

Im Unterschied zum Sinterverfahren, bei dem Stoffe in Pulverform unter Hitzeeinwirkung miteinander verbunden werden, geschieht dies beim SLS-Verfahren selektiv durch einen Laser (alternativ auch Elektronenstrahl oder Infrarotstrahl). Es wird also nur ein bestimmter Teil des Pulvers miteinander verschmolzen.

Dazu wird stets eine dünne Pulverschicht von der Beschichtungseinheit auf dem Druckbett ausgebracht. Der Laser (oder andere Energiequelle) wird nun punktgenau auf einzelne Stellen der Pulverschicht ausgerichtet, um die erste Schicht der Druckdaten auszubilden. Hierbei wird das Pulver an- oder aufgeschmolzen und verfestigt sich anschließend wieder durch geringfügiges Abkühlen. Das nicht aufgeschmolzene Pulver bleibt um die gesinterten Bereiche herum liegen und dient als Stützmaterial. Nachdem eine Schicht verfestigt ist, senkt sich das Druckbett um den Bruchteil eines Millimeters ab. Die Beschichtungseinheit fährt nun über das Druckbett und bringt die nächste Pulverschicht aus. Anschließend wird die zweite Schicht der Druckdaten durch den Laser (oder eine andere Energiequelle) gesintert. So entsteht schichtweise ein dreidimensionales Objekt.

### 3. Three-Dimensional Printing (3DP)

Das 3DP-Verfahren funktioniert sehr ähnlich wie das selektive Lasersintern, doch anstelle einer gerichteten Energiequelle verfährt ein Druckkopf über das Pulver. Dieser gibt winzige Tröpfchen von Bindemittel auf die zugrunde liegenden Pulverschichten ab, die so miteinander verbunden werden. Ansonsten ist dieses Verfahren dem SLS-Verfahren gleich.

### 4. Stereolithographie (SLA)

Anstelle eines Kunststoffdrahts oder Druckmaterials in Pulverform kommen beim Stereolithographie-Verfahren flüssige Harze, sog. Photopolymere, zum Einsatz. Sie werden schichtweise durch UV-Strahlung verhärtet und erzeugen so dreidimensionale Objekte. Dafür wird die Bauplattform im Harzbecken schrittweise abgesenkt. Es gibt auch Varianten (sog. Polyjet-Verfahren) ohne ein ganzes Becken mit flüssigem Harz. Dafür wird ein Epoxidharz tröpfchenweise aus einer Düse aufgebracht und durch einen UV-Laser sofort ausgehärtet.

### 5. Laminated Object Manufacturing (LOM)

### Alternativbezeichnung: Layer Laminated Manufacturing (LLM)

Das Verfahren basiert weder auf chemischen Reaktionen, noch auf einem thermischen Prozess. Es wird dabei mit einem trennenden Werkzeug (z.B. einem Messer oder Kohlendioxidlaser), einer Folie oder einer Platte (z.B. Papier) an der Kontur geschnitten und schichtweise aufeinander geklebt. So entsteht durch Absenken der Bauplattform ein Schichtobjekt aus geklebten, übereinanderliegenden Folien.

Eine oder mehrere wasserundurchlässige Schichten und/oder auch die Feuchteschicht können in derselben Art und/oder Dicke wie die Elektrode aufgebracht werden.

Gemäß zumindest einer Ausführungsform bedeckt die Feuchteschicht den Kondensator vollständig.

Dies kann heißen, dass die Feuchteschicht, nach außen, das heißt in der Querrichtung den Sensor nach außen abgrenzt und abschließt, sodass der Sensor zwischen der Feuchteschicht und dem Trägermaterial angeordnet ist.

Gemäß zumindest einer Ausführungsform weist der Sensor zumindest einen weiteren Kondensator auf, welcher in der Querrichtung unter oder über dem Kondensator angeordnet und durch eine weitere wasserundurchlässige Schicht beabstandet von dem Kondensator auf oder unter dieser weiteren wasserundurchlässigen Schicht angeordnet ist, sodass ein Kondensatorenstack entsteht.

Der weitere Kondensator kann in der gleichen Weise wie der Kondensator aufgebaut sein und ebenso in einer gleichen Weise wie der Kondensator auf die weitere wasserundurchlässige Schicht angeordnet sein.

Mittels eines derartigen Kondensatorenstacks kann die Sensorik ganz besonders einfach verfeinert werden nämlich insofern, als dass denkbar ist das bei zwei den Kondensatorstack ausbildenden Sensoren beide Sensoren die gleichen Aufgaben verrichten, jedoch durch die einzelnen Sensoren jeweilige Messwerte ermittelt werden, die zusammen genommen auf einen Mittelwert schließen lassen. Zum Beispiel wird von jedem der beiden Sensoren jeweils die (relative) Feuchtigkeit der Umgebung gemessen wobei aus diesen beiden Messwerten dann der Feuchtigkeitsmittelwert ermittelt wird. Gleiches kann entsprechend mit der Druckmessung geschehen, sodass die Genauigkeit der gesamten Messung insbesondere einer Kombination der Messungen von (relativer) Feuchtigkeit und dem jeweiligen Druck besonders genau ausgestaltet werden kann.

Gemäß zumindest einer Ausführungsform bildet die wasserundurchlässige Schicht und/oder die weitere wasserundurchlässige Schicht die dielektrische Sicht zumindest teilweise selbst aus.

Dies kann heißen, dass ein anstatt der separaten Positionierung einer dielektrischen Schicht neben der wasserundurchlässigen Schicht und/oder neben der weiteren wasserundurchlässigen Schicht, diese dielektrische Schicht selbst durch die wasserundurchlässige Schicht und/oder die weitere wasserundurchlässige Schicht gebildet ist.

Eine derartige Erzeugung der dielektrischen Schicht durch die wasserundurchlässigen Schicht(en) bildet daher ein besonders einfaches und kostengünstiges Herstellungsverfahren zu einer kostengünstigen Vorrichtung.

Davon abgesehen kann grundsätzlich vorgesehen sein die Elektroden, die dielektrische Schicht und die wasserundurchlässige Schicht(en) derart zueinander anzuordnen, dass ein elektrischer Kurzschluss in jedem Fall verhindert ist.

Gemäß zumindest einer Ausführungsform beträgt eine maximale Dicke der Feuchteschicht wenigstens 30% und höchstens 80% der maximalen Dicke der wasserundurchlässigen Schicht und/oder der maximalen Dicke der weiteren wasserundurchlässigen Schicht.

Dies stellt nicht nur einen besonders flach gebauten Sensor sicher, sondern gewährleistet auch eine besonders schnelle Reaktionszeit auf Feuchtigkeitsveränderungen. Die von außen auf die Feuchteschicht einwirkende Feuchtigkeit muss daher keine großen Strecken zu dem Dielektrikum durchwandern.

Im Folgenden wird die hier beschriebene Erfindung anhand zweier Ausführungsbeispiele und den dazugehörigen Figuren näher beschrieben.

Gleiche oder gleichwirkende Bestandteile sind mit den gleichen Bezugszeichen versehen.
Die Figuren 1A bis 1C zeigen ein Ausführungsbeispiel eines hier beschriebenen und erfindungsgemäßen Carsharing Verwaltungssystems.
In der Figur 2 ist einer schematisch perspektivischen Ansicht eine in Bezug auf die Schichtenordnung dargestellte Explosionszeichnung eines Sensors der hier beschriebenen Vorrichtung dargestellt.
In der Figur 3 ist ein weiteres Ausführungsbeispiel einer hier beschriebenen Vorrichtung gezeigt.
In der Figur 4 ist ein weiteres Ausführungsbeispiel einer hier beschriebenen Vorrichtung gezeigt.

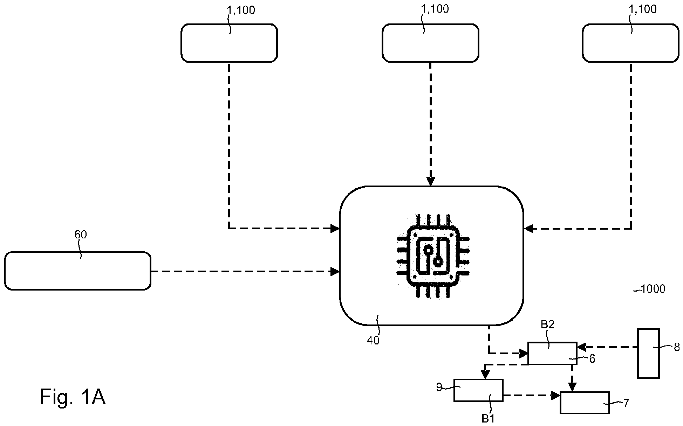

Der Figur 1A ist in einem Ausschnitt ein schematischer Aufbau eines hier beschriebenen erfindungsgemäßen Carsharing Verwaltungssystems 1000 gezeigt. Erkennbar ist eine Verarbeitungseinheit 5, welche in Datenkommunikation mit einer Mehrzahl von Sensoren 1 ist. Die Verarbeitungseinheit 5 bildet zusammen mit den Sensoren 1 eine Detektionsvorrichtung 100. Die von den einzelnen Sensoren 1 gemessen optischen und/oder physikalischen Werte und/oder Feuchtigkeits- und/oder Druckwerte werden an eine zentrale CPU 40 gesendet um dort gespeichert und/oder weiterverarbeitet zu werden. Zudem ist ein Temperatursensor 60, welcher eine Umgebungstemperatur und/oder eine Temperatur des Sensors 1 misst und an die Verarbeitungseinheit 4 der Vorrichtung 100 und/oder an die zentrale CPU 40 weiterleitet.

Die zentrale CPU 40 und/oder die Verarbeitungseinheit 5 vergleicht diese Daten, also die optischen und/oder physikalischen Werte und/oder Druck- und/oder Feuchtigkeitswerte, mit in einer Datenbank hinterlegten oder an die Datenbank gesendete entsprechenden Werten, und, vorzugsweise nur, bei Übereinstimmung mit diesen hinterlegten Werten oder entsprechenden Wertebereichen, die zentrale CPU 40 ein Freigabesignal an eine Abgleicheinrichtung 6 sendet, welche daraufhin ein Nutzungsobjekt 7 zur Nutzung freischaltet.

Eine Sendeeinrichtung 8, welche an die Abgleicheinrichtung 6 vorgegebene Werte oder Wertebereiche der optischen und/oder physikalischen Werte und/oder Druck- und/oder Feuchtigkeitswerte sendet ist vorhanden, wobei die Abgleicheinrichtung 6 diese empfangenen Werte mit den von der zentralen CPU 40 an die Abgleicheinrichtung 6 gesendeten Werte oder Wertebereiche vergleicht.

Auf Basis der einzelnen optischen und/oder physikalischen Werte und/oder Feuchte- und Druckwerte kann die die zentrale CPU 40 zumindest einen jeweiligen Kennwert ermitteln, aus welchem ableitbar ist, welches Individuum nach Gewicht und/oder Größe gerade die Vorrichtung 100 zur Messung von Druck und/oder Feuchtigkeit besetzt und/oder das Nutzungsobjekt 7 nutzt.

Außerdem ist eine Gesichtserfassungseinrichtung 9 dargestellt, mittels welcher die erfassten Bilddaten B1 mit den Berechtigungsdaten B2 verglichen werden, die ebenso in der Datenbank hinterlegt sind, wobei dieser Vergleich einen Klassifikationsvergleich von Datenklassen der Bilddaten B1 mit Datenklassen der Berechtigungsdaten B2 umfasst, wobei eine Klassifikation der Bilddaten B1 auf Basis von Bewegungsvektoren des Benutzers derart durchgeführt wird, dass zunächst aus der zeitlichen Bewegung des Benutzers ein Bewegungsprofil von der Gesichtserfassungseinrichtung 9 erstellt wird, wobei das Bewegungsprofil des Benutzers Bewegungsvektoren desselbigen umfasst, und weiter wobei Benutzer und/oder Benutzerbewegungen zu sogenannten Datenblops oder Datenclustern zusammengefasst werden, die dann in ihrer Form und/oder Ausdehnung klassifiziert werden.

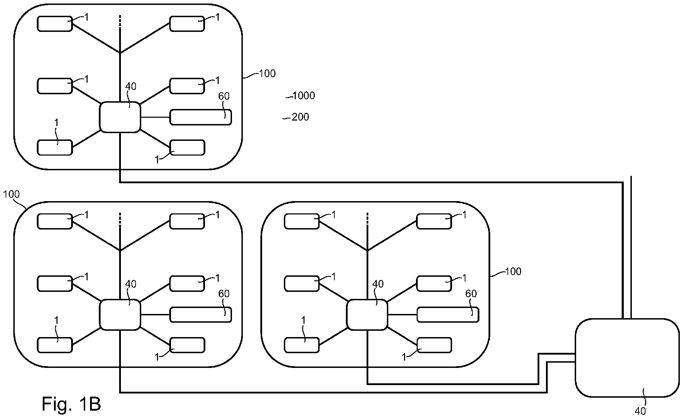

Die Figur 1B zeigt schematisch das gesamte Carsharing Verwaltungssystem 1000, mit einer Mehrzahl an Sensorgruppen, welche durch die einzelnen Detektionsvorrichtungen 100 zur Messung von optischen und/oder physikalischen Werten und/oder Druck und/oder Feuchtigkeit gebildet sind und welche jeweils eine Verarbeitungseinheit 5 zeigen. Jeder Verarbeitungseinheit 5 ist daher eine Mehrzahl an Sensoren 1 zugeordnet.

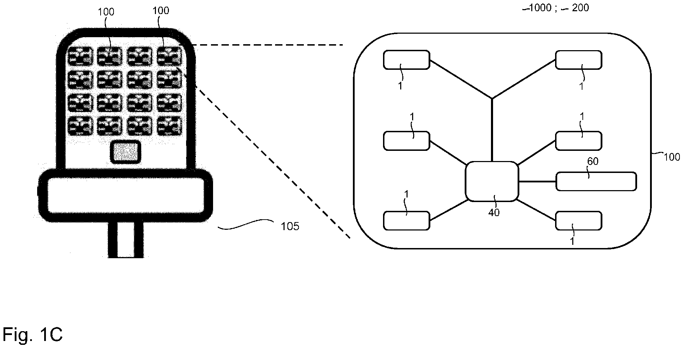

Die Figur 1C zeigt schematisch eine Verbauung und Integration des Carsharing Verwaltungssystems 1000 in einen Sitz, insbesondere in einen Fahrzeugsitz 105. Das Carsharing Verwaltungssystem 1000 kann daneben jedoch auch an einen Reifen des Nutzungsobjekts oder an einer anderen geeigneten Position an dem Nutzungsobjekt angeordnet sein, je nachdem, welche Werte bzw. Daten durch den Sensor erfasst werden sollen.

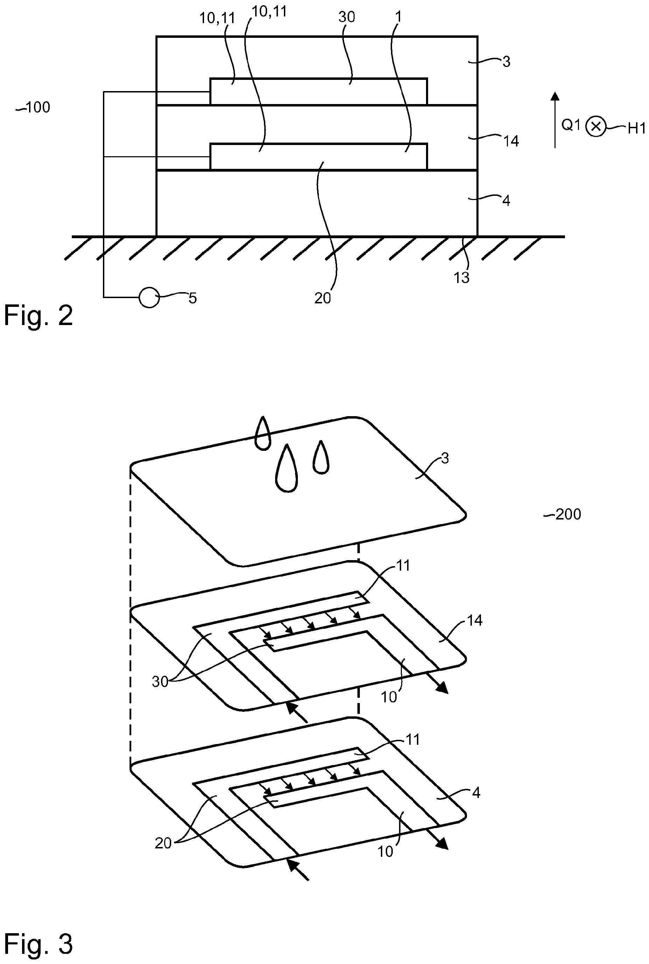

Wie nun der Figur 2 entnommen werden kann, ist dort beispielhaft eine Detektionsvorrichtung 100 zur Messung von Druck und/oder Feuchtigkeit gezeigt.

Beispielhaft ist dort ein Sensor 1 dargestellt, wobei der Sensor 1 einen Kondensatorstack zeigt mit einem Kondensator 20, sowie einem Kondensator 30, wobei die einzelnen Elektroden 10, 11 der Kondensatoren 20, 30 in der horizontalen Richtung H1 übereinander angeordnet sind, wobei alternativ hierzu jedoch selbstverständlich eine Anordnung der einzelnen Elektroden 10, 11 eines einzelnen Kondensators 20, 30 in der Querrichtung Q1 welche senkrecht zur horizontalen Richtung H1 verläuft und damit auch senkrecht zur Haupterstreckungsrichtung des dort gezeigten Sensors 1 verlaufen oder angeordnet sein können.

Die einzelnen Elektroden 10, 11 sind auf einem Trägermaterial 13 angeordnet. Bei dem Trägermaterial 13 kann es sich insbesondere um einen Webstoff, insbesondere um einen flexiblen Webstoff handeln.

Auf dem Trägermaterial 13 ist eine wasserundurchlässige Schicht 4 angeordnet, wobei auf dieser wasserundurchlässigen Schicht 4 die beiden Elektroden 10, 11 des Kondensators 20 in der horizontalen Richtung H1 aufgedruckt sind.

Die Elektroden 10, 11 des Kondensators 20 sind vollständig von einer weiteren wasserundurchlässigen Schicht 14 umgeben. Auf dieser wasserundurchlässigen Schicht 14 ist in der gleichen Art und Weise der weitere Kondensator 30 mit entsprechenden Elektroden 10, 11 aufgedruckt. Zudem sind in dem vorliegenden Ausführungsbeispiel freiliegende Außenflächen der einzelnen Elektroden 10, 11 des weiteren Kondensators 30 vorzugsweise vollständig von einer wasserdurchlässigen und/oder wasserabsorbierenden Feuchteschicht 3 umgeben.

Über diese Feuchteschicht 3 kann Wasser auf eine dielektrische Schicht 4 treffen, welche vorliegend in der horizontalen Richtung H1 zwischen den jeweiligen Elektroden 10, 11 eines Kondensators 20, 30 angeordnet ist.

Im vorliegenden Ausführungsbeispiel der Figuren 2 und 3 bildet die wasserundurchlässige Schicht 4 selbst eine dielektrische Schicht 4 des Kondensators 20 auf. Selbiges gilt für die weitere wasserundurchlässige Schicht 14 in Bezug auf den weiteren Kondensator 30.

Durch Auftreffen und Durchdringen der Feuchtigkeit über die Feuchteschicht 3 werden die dielektrischen Eigenschaften insbesondere der dielektrischen Schicht 2 des weiteren Kondensators 30 verändert.

Darüber hinaus ist eine Verarbeitungseinheit 5 erkennbar, welche in datentechnischer Verdingung mit den beiden Kondensatoren 20, 30 steht, wobei diese Verarbeitungseinheit 5 dazu eingerichtet und dafür vorgesehen ist, eine Änderung der relativen Feuchtigkeit der Umgebung und/oder der Feuchteschicht 3 zu messen.

Durch die in der Figur 3 dargestellte "stackwise"-Anordnung und dadurch dass die weitere wasserundurchlässige Schicht 14 verhindert, dass der Kondensator 20 mit Feuchtigkeit in Kontakt kommt kann daher vorgesehen sein, dass lediglich der weitere Kondensator 30 und dessen dielektrische Schicht 4 der Feuchtigkeit ausgesetzt ist. Hierzu kann die Verarbeitungseinheit 5 dann eine Veränderung der Kapazität des weiteren Kondensators 30 vergleichen mit der stabilen Kondensatorkapazität des Kondensators 20, sodass hierzu ein besonders einfacher Vergleich in der Veränderung der relativen Feuchtigkeit und/auch des jeweiligen Belastungsdruckes hergestellt sein kann.

Durch den in der Figur 2 dargestellten Pfeil ist auch eine Druckrichtung unter welcher der Sensor 1 mit Druck beaufschlagt wird gezeigt. Beides kann vorzugsweise durch den Sensor 1 und insbesondere durch die Detektionsvorrichtung 100 gemessen, ausgewertet und gespeichert werden. Hierzu dient insbesondere die in der Erfindung als wesentlich dargestellte Verarbeitungseinheit 5, welche zusätzlich auch entsprechende Druckwerte und insofern damit verbundene Änderungen in der Kapazität der einzelnen Sensoren 1 messen und auswerten kann, sodass die Verarbeitungseinheit 5 zusätzlich dazu eingerichtet und dafür vorgesehen ist eine durch äußeren Druck verursachte Kapazitätsänderung des Kondensators 20 und insbesondere auch des weiteren Kondensators 30 zu messen und/oder zu speichern.

Die Feuchteschicht 3 kann flexibel oder nicht flexibel ausgebildet sein. Zudem ist es möglich, dass die Feuchteschicht 3 als Webstoff ausgebildet ist. Insbesondere kann es sich um einen Webstoff handeln, welcher im einleitenden Teil der vorliegenden Anmeldung beispielhaft genannt wurde. Zudem ist es jedoch auch möglich dass es sich bei der Feuchteschicht 3 um ein Substrat handelt welches, zum Beispiel in Form eines Epitaxie- oder eines Klebeprozesses auf den weiteren Kondensator 30 aufgebracht, zum Beispiel aufgeklebt wurde.

Die wasserundurchlässige Schicht 14 und/oder die wasserundurchlässige Schicht 15 können ebenso flexibel und nicht flexibel, insbesondere auch ebenso in Form eines Webstoffes oder eines Substrats in der gleichen Weise wie die Feuchteschicht 3 ausgebildet sein.

Zudem ist vorteilhaft denkbar, dass die Elektroden 10, 11 der beiden Kondensatoren 20, 30 auf die wasserundurchlässige Schicht 14 und die weitere wasserundurchlässige Schicht 15 in Form eines Druckprozesses zum Beispiel eines Tintenstrahldruckprozesses aufgedruckt wurden.

In der Figur 3 ist eine Explosionszeichnung gezeigt, wobei insbesondere aus der Figur 3 die jeweilige Anordnung der Elektroden 10, 11 der Kondensatoren 20, 30 hervorgeht. Erkennbar ist wiederum die durch die Pfeilrichtung dargestellte Krafteinwirkung auf den Sensor 1, sowie durch die einzelnen schematisch dargestellten Tropfen einwirkende Feuchtigkeit. Insbesondere ist wiederum erkennbar, dass die Feuchtigkeit insbesondere zwischen den Elektroden 10, 11 eindringt und auf die jeweilige wasserdurchlässige Schicht 14 einen zum Beispiel erheblichen Effekt auf die elektrische Eigenschaft hat, sodass sich die Kapazität zumindest des weiteren Kondensators 30 wie in der Figur 1 erläutert jeweils ändert.

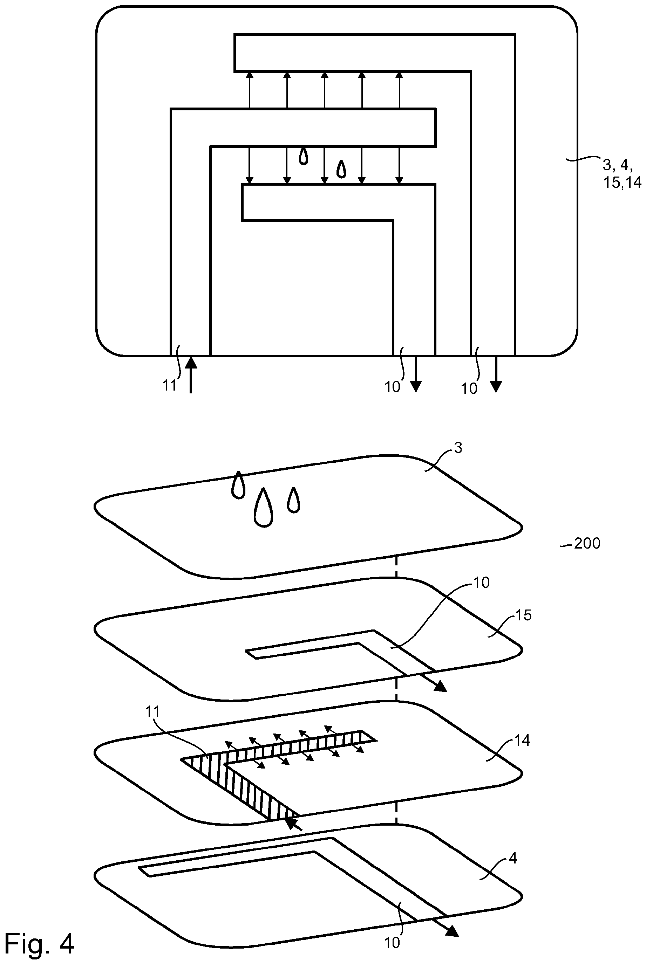

In der Figur 4 ist in einer weiteren Ausführungsform der hier beschriebenen Erfindung gezeigt, dass der Sensor 1 aus zwei Elektroden 10, sowie einer Elektrode 11 bestehen kann. Die Elektroden 10 haben eine Polarität (vorzugsweise die gleiche Polarität), während die Elektrode 11 eine davon unterschiedliche Polarität aufweist, wobei jedoch im unteren Teilbild der Figur 3 die Explosionszeichnung des linken Teils der Figur 3 gezeigt ist und erkennbar ist, dass drei wasserundurchlässige Schichten 4, 14, 15 verwendet werden. Die Elektroden 10 können auch unterschiedliche Polaritäten und/oder elektrische Potentiale aufweisen. Auch können die Elektroden 10 miteinander elektrisch verbunden sein.

Zum Beispiel können die Elektroden 10, 11 auch jeweils eine separate Polarität und/oder ein separates elektrisches Potential aufweisen und/oder generieren. Entsprechendes kann auch für die in den hier folgenden Figuren in Bezug auf die Elektroden gelten.

Zum Beispiel ist die unterste wasserundurchlässige Schicht wiederum die wasserundurchlässige Schicht 4, die darauffolgende wasserundurchlässige Schicht 14 und die in der Querrichtung Q1 darauf angeordnete wasserundurchlässige Schicht 15 eine weitere wasserundurchlässige Schicht, wobei jeweils eine Elektrode auf einer separaten wasserundurchlässigen Schicht jeweils aufgebracht insbesondere aufgedruckt ist.

In dieser Stackung der einzelnen wasserundurchlässigen Schichten 4, 14 und 15 und wird daher durch Zusammenführen dieser Schichten der im linken Teil der Figur 4 gezeigte Kondensator 20 erzeugt, wobei hierbei in der Querrichtung Q1, die Elektroden 10 jeweils, wie im dementsprechenden Teilbild entnommen werden kann auf unterschiedlichen Ebenen angeordnet sin.

Alternativ hierzu kann auch die Elektrode 11 zusammen mit zumindest einer der Elektroden 10 in einer gemeinsamen Ebene, das heißt auf oder in einer gemeinsamen wasserundurchlässigen Schicht 4, 14, 15 aufgebracht werden, sodass zum Beispiel nur noch die zweite der Elektroden 10 auf eine separaten wasserundurchlässigen Schicht 4, 14, 15 aufgestackt werden muss.

Grundsätzlich können daher die einzelnen Elektroden 10,11 in unterschiedlichen Ebenen in der Q1-Richtung zueinander angeordnet sein. Zum Beispiel gilt eine paarweise Zuordnung zwischen genau einer wasserundurchlässigen Schicht 4, 14, 15 mit genau einer Elektrode 10, 11.

Die Erfindung ist nicht durch die Beschreibung anhand des Ausführungsbeispiels beschränkt. Vielmehr umfasst die Erfindung jedes neue Merkmal, sowie jede Kombination von Merkmalen was insbesondere jede Kombination von Merkmalen in den Patentansprüchen beinhaltet, auch wenn dieses Merkmal oder diese Kombination selbst nicht explizit in den Patentansprüchen oder in den Ausführungsbeispielen angegeben ist.

### Bezugszeichenliste

- 1: Sensor
- 3: Feuchteschicht
- 4: dielektrische Schicht/ wasserundurchlässige Schicht
- 5: Verarbeitungseinheit
- 6: Abgleicheinrichtung
- 7: Nutzungsobjekt
- 8: Sendeeinrichtung
- 9: Gesichtserfassungseinrichtung
- 10: Elektrode
- 11: Elektrode
- 12: Elektrode
- 13: Trägermaterial
- 14: wasserundurchlässige Schicht
- 15: wasserundurchlässige Schicht
- 20: Kondensator
- 30: Kondensator
- 40: CPU
- 60: Temperatursensor
- 100: Detektionsvorrichtung
- 105: Fahrzeugsitz
- 1000: Carsharing Verwaltungssystem

- B1: Bilddaten
- B2: Berechtigungsdaten
- H1: horizontalen Richtung
- Q1: Querrichtung

## Patentansprüche

1. Carsharing Verwaltungssystem (1000) zur Verwaltung von Nutzungsobjekten, insbesondere von Fahrzeugen, umfassend zumindest eine
- an dem Nutzungsobjekt verbaute Detektionsvorrichtung (100) zur Identifikation des jeweils in einer Datenbank hinterlegten Nutzers, umfassend
∘ zumindest einen optischen und/oder physikalischen Sensor (1), der den Nutzer sensorisch detektiert und/oder optische Daten gewinnt sowie zumindest eine Verarbeitungseinheit (5), welche dazu eingerichtet und dafür vorgesehen ist Messwerte des Sensors (1) zu speichern, wobei von der Verarbeitungseinheit (5) die von dem Sensor gemessenen Daten an eine zentrale CPU (40) gesendet werden, wobei diese Daten von der Verarbeitungseinheit (5) verarbeitet werden,
**dadurch gekennzeichnet, dass**
die zentrale CPU (40) und/oder die Verarbeitungseinheit (5) diese Daten, mit in einer Datenbank hinterlegten oder an die Datenbank gesendete entsprechenden Werten vergleicht, und, vorzugsweise nur, bei Übereinstimmung mit diesen hinterlegten Werten oder entsprechenden Wertebereichen, die zentrale CPU (40) ein Freigabesignal an eine Abgleicheinrichtung (6) sendet, welche daraufhin ein Nutzungsobjekt (7) zur Nutzung freischaltet, insbesondere wobei die zentrale CPU (40) abseits von dem Nutzungsobjekt (7) in zumindest einer, insbesondere ortsfesten, Montagestation verbaut ist, sodass die zentrale CPU (40) drahtlos mit der Abgleicheinrichtung (6) kommunizieren kann, und weiter wobei dass das Verwaltungssystem zumindest einen Temperatursensor aufweist, wobei der Temperatursensor eine Umgebungstemperatur und/oder eine Temperatur eines Sensors und/oder eine Temperatur eine Benutzers des Nutzungsobjekts misst und diesen Wert an die zentrale CPU weiterleitet, insbesondere wobei auf Basis des gemessenen Temperaturwertes des Benutzer eindeutig auf die Identität des Benutzers geschlossen werden kann.

2. Carsharing Verwaltungssystem (1000) zur Verwaltung von Nutzungsobjekten nach Anspruch 1, insbesondere von Fahrzeugen,
**dadurch gekennzeichnet, dass**
Benutzerdaten, wie Größe, Gewicht und/oder Alter des Benutzers des Nutzungsobjektes, in der zentralen CPU (40) hinterlegt sind.

3. Carsharing Verwaltungssystem (1000) zur Verwaltung von Nutzungsobjekten nach Anspruch 1, insbesondere von Fahrzeugen,
**dadurch gekennzeichnet, dass**
der Temperatursensor innerhalb des Nutzungsobjektes, vorzugsweise in einer Fahrzeugkanzel verbaut ist, und von dort aus die Temperatur, insbesondere, durch eine Infrarot Wärmemessung, misst.

4. Carsharing Verwaltungssystem (1000) zur Verwaltung von Nutzungsobjekten nach Anspruch 1, insbesondere von Fahrzeugen,
**dadurch gekennzeichnet, dass**
es sich bei dem Temperatursensor um den Sensor (1) handelt oder aber die beiden Sensoren verschieden voneinander sind.

5. Carsharing Verwaltungssystem (1000) nach zumindest einem der vorhergehenden Ansprüche,
**dadurch gekennzeichnet, das**
die Abgleicheinrichtung (6) an dem Nutzungsobjekt verbaut ist und sich damit mit dem Nutzungsobjekt mitbewegt.

6. Carsharing Verwaltungssystem (1000) nach zumindest einem der vorhergehenden Ansprüche,
**dadurch gekennzeichnet, das**
es sich bei dem Sensor um einen Infrarot-, Ultraschall-, UV,- Sensor, einen Laser, einen biometrischen Sensor, Drucksensor, Feuchtigkeitssensor, Temperatur und/oder um eine optische Bildkamera handelt.

7. Carsharing Verwaltungssystem (1000) nach zumindest einem der vorhergehenden Ansprüche,
**dadurch gekennzeichnet, das**
- es sich bei dem Sensor (1) um einen Sensor zur Messung von Druck und/oder Feuchtigkeit handelt, wobei der Sensor (1)
- zumindest einen Kondensator (20) mit zumindest zwei Elektroden (10, 11), welche, insbesondere in einer horizontalen Richtung (H1) entlang eines und auf einem, insbesondere flexiblem, Trägermaterial (13) zueinander angeordnet sind, wobei zwischen den Elektroden (10, 11) zumindest eine dielektrische Schicht (4) angeordnet ist, wobei
auf einer dem Trägermaterial (13) abgewandten Seite zumindest einer Elektrode (10, 11) und/oder der dielektrischen Schicht (2) zumindest stellenweise zumindest eine zumindest teilweise flüssigkeitsdurchlässige und/oder flüssigkeitsabsorbierende Feuchteschicht (3) angeordnet ist, wobei somit die zumindest eine Elektrode (10, 11) und/oder die dielektrische Schicht (4) in einer Querrichtung (Q1) zwischen dem Trägermaterial (13) und der Feuchteschicht (3) angeordnet sind, sodass sich eine Kapazität durch die auf die dielektrische Schicht (4) zumindest teilweise treffende Flüssigkeit zumindest teilweise verändert, wobei die Verarbeitungseinheit (5) dazu eingerichtet und dafür vorgesehen ist Messwerte des Sensors (1) zu messen und/oder zu speichern, sodass ein kapazitiver Feuchtesensor entsteht, wobei von der Verarbeitungseinheit (5) die von dem Sensor gemessenen Daten an die zentrale CPU (40) gesendet werden, wobei diese Daten von der Verarbeitungseinheit (5) verarbeitet werden.

8. Carsharing Verwaltungssystem (1000) nach zumindest einem der vorhergehenden Ansprüche,
**gekennzeichnet, durch**
zumindest eine Sendeeinrichtung (8), welche an die Abgleicheinrichtung (6) vorgegebene Werte oder Wertebereiche der Druck- und/oder Feuchtigkeitswerte und/oder Temperaturwerte und/oder optischen gewonnenen Werte sendet, und die Abgleicheinrichtung (6) diese empfangenen Werte mit den von der zentralen CPU (40) an die Abgleicheinrichtung (6) gesendeten Werte oder Wertebereiche vergleicht.

9. Carsharing Verwaltungssystem (1000) nach dem vorhergehenden Anspruch
**dadurch gekennzeichnet, dass**
es sich bei der Sendeeinrichtung (8) um einen Fahrzeugschlüssel handelt oder diese zumindest ein Teil davon ist, wobei die Sendeeinrichtung (8) dazu eingerichtet und dafür vorgesehen ist mittels einer drahtlosen Verbindung mit der Abgleicheinrichtung (6) und/oder der zentralen CPU (40) zu kommunizieren.

10. Carsharing Verwaltungssystem (1000) nach zumindest einem der vorhergehenden Ansprüche,
**dadurch gekennzeichnet, dass**
die Abgleicheinrichtung (6) in den Fahrzeugschlüssel integriert verbaut ist.

11. Carsharing Verwaltungssystem (1000) nach zumindest einem der vorhergehenden Ansprüche,
**dadurch gekennzeichnet, dass**
die Abgleicheinrichtung (6) ein Fahrzeugschloss, eine Wegfahrsperre, ein Lenkradschloss, ein Gas- und/oder Bremspedal zur Nutzung bei einer Datenübereinstimmung freigibt.

12. Carsharing Verwaltungssystem (1000) nach zumindest einem der vorhergehenden Ansprüche,
**dadurch gekennzeichnet, dass**
auf Basis der einzelnen Werte die zentrale CPU (40) zumindest einen jeweiligen Kennwert ermittelt, aus welchem ableitbar ist, welches Individuum nach Gewicht und/oder Größe und/oder Körperabdruck gerade die Vorrichtung (100) zur Messung von Druck und/oder Feuchtigkeit besetzt, und/oder dass nach einer Sitzbelegung des Fahrzeugsitzes abhängig von der identifizierten Person eine vorgespeicherte Sitzposition, ein vorgespeichertes Licht- und /oder Schallambiente und/oder ein Klima, ein vorgespeicherter Musik- und/oder Radiosender oder andere personalisierte Dienste ausgewählt und/oder angewendet werden.

13. Carsharing Verwaltungssystem (1000) nach Anspruch 1,
**dadurch gekennzeichnet, dass**
sofern die gemessenen Daten mit den hinterlegten Daten nicht übereinstimmen und/oder um mehr als 25% von den hinterlegten Datenwerten abweichen eine Gesichtserfassungseinrichtung (9) und/oder Körperabdruckerfassungseinrichtung ein Gesicht und/oder einen Körperabdruck des Benutzers auf Basis von Bilddaten (B1), welche den optischen gewonnenen Daten entsprechen oder aus diesen gewonnen sind, erfasst, und trotz der Datenabweichung dann das Nutzungselement freischaltet, sofern die Gesichtserfassungseinrichtung (9) ein Benutzergesicht als identisch mit einem in der Gesichtserfassungseinrichtung (9) hinterlegten Gesicht identifiziert.

14. Carsharing Verwaltungssystem (1000) nach zumindest einem der vorhergehenden Ansprüche,
**dadurch gekennzeichnet, dass**
die erfassten Bilddaten (B1) mit den Berechtigungsdaten (B2) verglichen werden, die ebenso in der Datenbank hinterlegt sind, wobei dieser Vergleich einen Klassifikationsvergleich von Datenklassen der Bilddaten (B1) mit Datenklassen der Berechtigungsdaten (B2) umfasst, wobei eine Klassifikation der Bilddaten (B1) auf Basis von Bewegungsvektoren des Benutzers derart durchgeführt wird, dass zunächst aus der zeitlichen Bewegung des Benutzers ein Bewegungsprofil von der Gesichtserfassungseinrichtung (9) erstellt wird, wobei das Bewegungsprofil des Benutzers Bewegungsvektoren desselbigen umfasst, und weiter wobei
Benutzer und/oder Benutzerbewegungen zu sogenannten Datenblops oder Datenclustern zusammengefasst werden, die dann in ihrer Form und/oder Ausdehnung klassifiziert werden.
